# EUROPEAN PATENT SPECIFICATION

(11) **EP 3 800 199 B1**
(45) Date of publication and mention of the grant of the patent: **21.08.2024**
(21) Application number: 20197179.3
(22) Date of filing: 04.05.2016
(51) Int. Cl.: C07K 14/47, C07K 16/18, G01N 33/574, A61P 35/00, C07K 14/725, C12N 5/0783, G01N 33/50, A61K 39/00

(54) **H3.3 CTL PEPTIDES AND USES THEREOF**
H3.3-CTL-PEPTIDE UND VERWENDUNGEN DAVON
PEPTIDES H3.3 CTL ET LEURS UTILISATIONS

(30) Priority: 05.05.2015 US 201562157362 P; 31.08.2015 US 201562212508 P
(43) Date of publication of application: 07.04.2021
(62) Divisional of application: 16790043.0
(73) Proprietor: The Regents of the University of California, Oakland, CA 94607 (US)
(72) Inventor: OKADA, Hideo, Oakland, CA 94607 (US); HOU, Yafei, Oakland, CA 94607 (US)
(74) Representative: J A Kemp LLP

(56) References cited:
- WO-A1-2012/038055
- WO-A1-2013/075237
- WO-A2-2009/147201
- US-A1- 2007 044 171
- US-A1- 2014 107 039
- P. W. LEWIS ET AL: "Inhibition of PRC2 Activity by a Gain-of-Function H3 Mutation Found in Pediatric Glioblastoma", SCIENCE, vol. 340, no. 6134, 17 May 2013 (2013-05-17), pages 857 - 861, XP055253687, ISSN: 0036-8075, DOI: 10.1126/science.1232245
- CUTHBERT G L ET AL: "Histone deimination antagonizes arginine methylation", CELL, CELL PRESS, AMSTERDAM, NL, vol. 118, no. 5, 3 September 2004 (2004-09-03), pages 545 - 553, XP002358960, ISSN: 0092-8674, DOI: 10.1016/J.CELL.2004.08.020
- KOVALIC D K ET AL: "Wheat recombinant protein SEQ ID NO 152042", GENESEQ,, 22 February 2007 (2007-02-22), XP002751291
- DATABASE Geneseq [online] 10 May 2012 (2012-05-10), "Cytomegalovirus phosphoprotein 65 specific TCR alpha chain, SEQ ID 10.", XP002786132, retrieved from EBI accession no. GSP:AZU40581 Database accession no. AZU40581
- M. I. VANAN ET AL: "Management of high-grade gliomas in the pediatric patient: Past, present, and future", NEURO-ONCOLOGY PRACTICE, vol. 1, no. 4, 12 September 2014 (2014-09-12), pages 145 - 157, XP055519137, ISSN: 2054-2577, DOI: 10.1093/nop/npu022
- T FUKAZAWA ET AL: "Testing the importance of each residue in a HLA-B27-binding peptide using monoclonal antibodies", THE JOURNAL OF IMMUNOLOGY, 1 February 1994 (1994-02-01), United States, pages 1190 - 1196, XP055518741, Retrieved from the Internet <URL:http://www.jimmunol.org/content/152/3/1190.full-text.pdf>
- YAFEI HOU ET AL: "Novel and shared neoantigen for glioma T cell therapy derived from histone 3 variant H3.3 K27M mutation", JOURNAL FOR IMMUNOTHERAPY OF CANCER, BIOMED CENTRAL LTD, LONDON, UK, vol. 3, no. 2, 4 November 2015 (2015-11-04), pages 1 - 2, XP021235622, DOI: 10.1186/2051-1426-3-S2-P445
- ZINAL S. CHHEDA ET AL: "Novel and shared neoantigen derived from histone 3 variant H3.3K27M mutation for glioma T cell therapy", THE JOURNAL OF EXPERIMENTAL MEDICINE, vol. 215, no. 1, 4 December 2017 (2017-12-04), US, pages 141 - 157, XP055585208, ISSN: 0022-1007, DOI: 10.1084/jem.20171046

## Description

This invention was made with government support under grant no. R21 NS083171 awarded by the National Institutes of Health. The government has certain rights in the invention.

### BACKGROUND OF THE INVENTION

Malignant gliomas, including glioblastoma (GBM) and diffuse intrinsic pontine gliomas (DIPG), are lethal brain tumors in both adults and children. Recent genetic studies have revealed that malignant gliomas in children and young adults often show recurrent missense mutations in *H3F3A,* which encodes the replication-independent histone 3 variant H3.3. *See, e.g.,* Lewis et al., Science Vol. 340 no. 6134 pp. 857-861 (2013). Approximately 30% of overall GBM and 70% of DIPG cases harbor the amino-acid substitution from lysine (K) to methionine (M) at the position 27 of H3.3 (K27M mutation, hereafter), which is universally associated with shorter survival in DIPG patients compared with patients with non-mutated H3.3. The adaptive immune system, such as T lymphocytes (T cells hereafter), are normally tolerant to normal self-proteins, but can recognize mutated amino-acids as non-self. Hence cancer-specific mutations can be suitable targets of cancer immunotherapy, such as cancer vaccines and adoptive T cell transfer therapy.

US 2014/107039 describes Polycomb Repressive Complex 2 (prc2) inhibitors and uses thereof. WO 2013/075237 describes mutations of histone proteins associated with proliferative disorders. P. W. Lewis et al., Science, 2013, 340:857-861 describe inhibition of PRC2 activity by a gain-of-function H3 mutation found in pediatric glioblastoma. Cuthbert G. L. et al., Cell, 2004, 118(5):545-553 describe that histone deamination antagonizes arginine methylation.
WO 2009/147201 describes specific binding molecules directed against citrulline-containing epitopes for use in the therapy and prevention of inflammatory conditions. Kovalic D. K. et al. (Geneseq, 2007) and US 2007/044171 describe the wheat recombinant protein "SEQ ID NO 152042". Database Geneseq, 2012 (EBI accession no. GSP:AZU40581) and WO 2012/038055 describe the Cytomegalovirus phosphoprotein 65 specific TCR alpha chain "SEQ ID 10". Vanan M. I. et al., Neruo-oncology Practice, 2014, 1(4):145-157 describes the management of high-grade gliomas in the pediatric patient. Fukazawa T. et al., The Journal of Immunology, 1994, 1190-1196 describes testing the importance of each residue in a HLA-B27-binding peptide using monoclonal antibodies.

### SUMMARY OF THE INVENTION

The invention provides a modified T-cell comprising a T-cell receptor (TCR) specific for a replication-independent histone 3 variant H3.3 or H3.1 peptide comprising the amino acid sequence (R/A)MSAP(S/A)TGGV (SEQ ID NO: 1), wherein the TCR comprises: (a) a TCR alpha chain, comprising complementarity-determining regions (CDRs) 1, 2, and 3 comprising SEQ ID NOs: 12, 13, and 14, respectively; and (b) a TCR beta chain, comprising CDRs 1, 2, and 3 comprising SEQ ID NOs: 15, 16, and 17, respectively; wherein the TCR binds the histone 3 variant H3.3 or H3.1 peptide when the peptide is in a complex with a major histocompatibility complex (MHC).

### Further disclosure

Described herein is an isolated peptide consisting of less than 100, 75, 50, or 30 amino acids is described, wherein the peptide may comprise (R/A)MSAP(S/A)TGGV (SEQ ID NO:1). The peptide may consist of 10-14 amino acids. The peptide may consist of (R/A)MSAP(S/A)TGGV (SEQ ID NO:1). The R in SEQ ID NO:1 may be citrullinated. The R in SEQ ID NO: 1 may not be citrullinated.

Also described herein is a fluorochrome-conjugated peptide-major histocompatibility complex (pMHC) multimer, wherein the peptide may be as described above or elsewhere herein.

Also described herein is a nucleic acid, optionally isolated or purified, encoding the peptide as described above or elsewhere herein. The nucleic acid may be a plasmid or a viral vector. The vector may be capable of delivering the nucleic acid into an antigen presenting cell (APC).

Also described herein is a cell comprising a heterologous peptide consisting of 10-12 or 10-14 *(e.g.,* 10, 11, 12, 13, or 14) amino acids, wherein the peptide may comprise (R/A)MSAP(S/A)TGGV (SEQ ID NO: 1). The peptide may consist of (R/A)MSAP(S/A)TGGV (SEQ ID NO: 1). The R in SEQ ID NO: 1 may be citrullinated. The R in SEQ ID NO: 1 may not be citrullinated. The cell may be an antigen presenting cell (APC). The cell may be a bacterial cell. The bacterial cell may be *E. coli* or *Listeria monocytogenes.* The APC may present the heterologous peptide on the surface of the cell. The APC may comprise a heterologous expression cassette comprising a promoter operably linked to a polynucleotide encoding the peptide.

Also described herein is a method of inducing an immune response in a human individual. The method may comprise administering a sufficient amount of the APC as described above or elsewhere herein to the human individual, thereby inducing an immune response in the human individual to replication-independent histone 3 variant H3.3 or H3.1. The APC may be from the individual (autologous). the immune response may be a cytotoxic T-cell response. The human individual may have a glioma. The individual may carry an HLA-2*201 allele.

Also described herein is a composition for stimulating an immune response to replication- independent histone 3 variant H3.3, the composition comprising a peptide consisting of 10-12 or 10-14 (*e.g.,* 10, 11, 12, 13, or 14) amino acids, wherein the peptide comprises (R/A)MSAP(S/A)TGGV (SEQ ID NO: 1). The composition may comprise a peptide consisting of (R/A)MSAP(S/A)TGGV (SEQ ID NO:1). The composition may further comprise an adjuvant. The R in SEQ ID NO: 1 may be citrullinated. The R in SEQ ID NO: 1 may not be citrullinated.

Also described herein is a method of inducing an immune response in a human individual, the method comprising administering a sufficient amount of the composition as described above or elsewhere herein to the human individual, thereby inducing an immune response in the human individual to histone 3 variant H3.3 or H3.1. The composition may comprise an adjuvant selected from the group consisting of polyICLC and Bacillus Calmette-Guérin (BCG) vaccine. The immune response may be a cytotoxic T-cell response. The human individual may have a glioma. The individual may carry an HLA-2*201 allele.

Also described is an antibody that specifically binds to RMSAPSTGGV (SEQ ID NO:2) and does not bind to RKSAPSTGGV (SEQ ID NO:3). The antibody may be linked to a heterologous detectable label. The label may be fluorescent.

Also described herein is a T-cell expressing a polypeptide or polypeptides comprising a T-cell receptor (TCR), or a peptide/MHC complex-binding fragment thereof, may bind the peptide as described above or elsewhere herein in a peptide/MHC complex. The TCR may be heterologous to the T-cell. Expression of the TCR may be under the control of a heterologous promoter (e.g., as transgenes). The TCR may comprise one or more of the CDRs as listed in SEQ ID NOs: 12-17, optionally in a heterologous framework region. The TCR will in general be formed of an alpha and a beta chain (two separate polypeptides). The TCR may comprise SEQ ID NO:8, SEQ ID NO: 10, or both, either as a fusion protein or as separate proteins.

Also described herein is a method of enriching for T-cells expressing a TCR that binds the peptide as described above or elsewhere herein. The method may comprise generating a starting culture of T-cells expressing TCRs; culturing the T-cells in the presence of the peptide to generate an enriched culture of T-cells, wherein the enriched culture is enriched for T-cells expressing TCRs that bind the peptide compared to the starting culture. The culturing may comprise culturing the T-cells in the presence of IL-2, IL-4, IL-7, IL-15, or combinations thereof. The method may further comprise sorting cells in the enriched culture for T-cells that bind the peptide in a peptide/MHC complex to form a further enriched population of T-cells expressing TCRs that bind the peptide/MHC complex. The sorting may comprise contacting cells in the enriched culture with a fluorochrome-conjugated peptide-major histocompatibility complex (pMHC) multimer.

### DEFINITIONS

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (*e*.*g*., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzev et al. Nucleic Acid Res. 19:5081 (1991); Ohtsuka et al., J. Biol. Chem. 260:2605-2608 (1985); Rossolini et al., Mol. Cell. Probes 8:91-98 (1994)).

The terms "polypeptide", "peptide" and "protein" are used interchangeably herein to refer to a polymer of amino acid residues. The terms encompass amino acid polymers in which one or more amino acid residue is an artificial chemical mimetic of a corresponding naturally occurring amino acid, as well as naturally occurring amino acid polymers and non-naturally occurring amino acid polymers.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e*.*g*., hydroxyproline, γ-carboxyglutamate, and O-phosphoserine. The term "amino acid analogs" refers to compounds that have the same basic chemical structure as a naturally occurring amino acid, *i.e.,* an a carbon that is bound to a hydrogen, a carboxyl group, an amino group, and an R group, *e*.*g*., homoserine, norleucine, methionine sulfoxide, methionine methyl sulfonium. Such analogs have modified R groups (*e*.*g*., norleucine) or modified peptide backbones, but retain the same basic chemical structure as a naturally occurring amino acid. The term "amino acid mimetics" refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and UGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

For amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles described herein.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) *(see, e.g.,* Creighton, Proteins (1984)).

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (*e*.*g*., as commonly used in an ELISA), biotin, digoxigenin, or haptens and proteins which can be made detectable, *e*.*g*., by incorporating a radiolabel into the peptide or used to detect antibodies specifically reactive with the peptide.

An antibody can consist of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. An "antibody" functions as a binding protein and is structurally defined as comprising an amino acid sequence from or derived from the framework region of an immunoglobulin-encoding gene of a vertebrate animal.

A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

The term "antibody" as used herein encompasses antibody fragments that retain antigen-binding specificity. For example, there are a number of well characterized antibody fragments. Thus, for example, pepsin digests an antibody C-terminal to the disulfide linkages in the hinge region to produce F(ab)'2, a dimer of Fab which itself is a light chain joined to VH-CH1 by a disulfide bond. The F(ab)'2 may be reduced under mild conditions to break the disulfide linkage in the hinge region thereby converting the (Fab')2 dimer into an Fab' monomer. The Fab' monomer is essentially an Fab with part of the hinge region (see, *e*.*g*., Fundamental Immunology, W.E. Paul, ed., Raven Press, N.Y. (1993), for a more detailed description of other antibody fragments). While various antibody fragments are defined in terms of the digestion of an intact antibody, one of skill will appreciate that fragments can be synthesized de novo either chemically or by utilizing recombinant DNA methodology. Thus, the term antibody, as used herein also includes antibody fragments either produced by the modification or digestion of whole antibodies or synthesized using recombinant DNA methodologies.

Antibodies can include VH-VL dimers, including single chain antibodies (antibodies that exist as a single polypeptide chain), such as single chain Fv antibodies (sFv or scFv) in which a variable heavy and a variable light region are joined together (directly or through a peptide linker) to form a continuous polypeptide. The single chain Fv antibody is a covalently linked VH-VL which may be expressed from a nucleic acid including VH- and VL- encoding sequences either joined directly or joined by a peptide-encoding linker (*e*.*g*., Fluston, et al. Proc. Nat. Acad. Set. USA, 85:5879-5883, 1988). While the VH and VL are connected to each as a single polypeptide chain, the V_{H} and VL domains associate non- covalently. Alternatively, the antibody can be another fragment. Other fragments can also be generated, *e*.*g*., using recombinant techniques, as soluble proteins or as fragments obtained from display methods. Antibodies can also include diantibodies and miniantibodies. Antibodies may also include heavy chain dimers, such as antibodies from camelids. Thus, an antibody may be dimeric. The antibody may be in a monomeric form that has an active isotype. The antibody may be in a multivalent form, *e.g.,* a trivalent or tetravalent form.

As used herein, "complementarity-determining region (CDR)" refers to the three hypervariable regions in each chain that interrupt the four "framework" regions established by the light and heavy chain variable regions. The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a VH CDR3 is located in the variable domain of the heavy chain of the antibody in which it is found, whereas a V_{L} CDR1 is the CDR1 from the variable domain of the light chain of the antibody in which it is found.

As used herein, "V-region" refers to an antibody variable region domain comprising the segments of Framework 1, CDR1, Framework 2, CDR2, and Framework3, including CDR3 and Framework 4, which segments are added to the V-segment as a consequence of rearrangement of the heavy chain and light chain V-region genes during B-cell differentiation.

The sequences of the framework regions of different light or heavy chains are relatively conserved within a species. The framework region of an antibody, that is the combined framework regions of the constituent light and heavy chains, serves to position and align the CDRs in three dimensional space.

The amino acid sequences of the CDRs and framework regions can be determined using various well known definitions in the art, *e*.*g*., Kabat, Chothia, international ImMunoGeneTics database (IMGT), and AbM (*see, e.g.,* Johnson *et al., supra;* Chothia & Lesk, 1987, Canonical structures for the hypervariable regions of immunoglobulins. J. Mol. Biol. 196, 901-917; Chothia C. et al., 1989, Conformations of immunoglobulin hypervariable regions. Nature 342, 877-883; Chothia C. et al., 1992, structural repertoire of the human VH segments J. Mol. Biol. 227, 799-817; Al-Lazikani et al., J.Mol.Biol 1997, 273(4)). Definitions of antigen combining sites are also described in the following: Ruiz et al., IMGT, the international ImMunoGeneTics database. Nucleic Acids Res., 28, 219-221 (2000); and Lefranc, M.-P. IMGT, the international ImMunoGeneTics database. Nucleic Acids Res. Jan 1;29(1):207-9 (2001); MacCallum et al, Antibody-antigen interactions: Contact analysis and binding site topography, J. Mol. Biol., 262 (5), 732-745 (1996); and Martin et al, Proc. Natl Acad. Sci. USA, 86, 9268-9272 (1989); Martin, et al, Methods Enzymol., 203, 121-153, (1991); Pedersen et al, Immunomethods, 1, 126, (1992); and Rees et al, In Sternberg M.J.E. (ed.), Protein Structure Prediction. Oxford University Press, Oxford, 141-172 1996).

"Epitope" or "antigenic determinant" refers to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. *See, e.g.,* Epitope Mapping Protocols in Methods in Molecular Biology, Vol. 66, Glenn E. Morris, Ed (1996).

As used herein, "chimeric antibody" refers to an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, *e*.*g*., an enzyme, toxin, hormone, growth factor, drug, etc.; or (b) the variable region, or a portion thereof, is altered, replaced or exchanged with a variable region, or portion thereof, having a different or altered antigen specificity; or with corresponding sequences from another species or from another antibody class or subclass.

The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologics such as a mixture of cells or a cell lysate. Thus, under designated immunoassay conditions, the specified antibodies bind to a particular protein (*e*.*g*., SEQ ID NO:2) at least two times the background and more typically more than 10 to 100 times background. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, Using Antibodies, A Laboratory Manual (1998) for a description of immunoassay formats and conditions that can be used to determine specific immunoreactivity).

"Antigen presenting cells", or "APCs" are cells that cells that mediate the cellular immune response by processing and presenting antigens to the T-cell receptor and include Langerhans cells, veiled cells of afferent lymphatics, dendritic cells and interdigitating cells of lymphoid organs. APCs include mononuclear cells such as lymphocytes and macrophages.

A polynucleotide or polypeptide sequence is "heterologous" to an organism or a second polynucleotide sequence if it originates from a foreign species, or, if from the same species, is modified from its original form. For example, when a heterologous promoter is said to be operably linked to a coding sequence, it means that the coding sequence is derived from one species whereas the promoter sequence is derived from another, different species; or, if both are derived from the same species, the coding sequence is not naturally associated with the promoter (*e.g.,* the promoter is a genetically engineered promoter or promoter fragment not found naturally associated with the coding sequence).

### BRIEF DESCRIPTION OF THE DRAWINGS

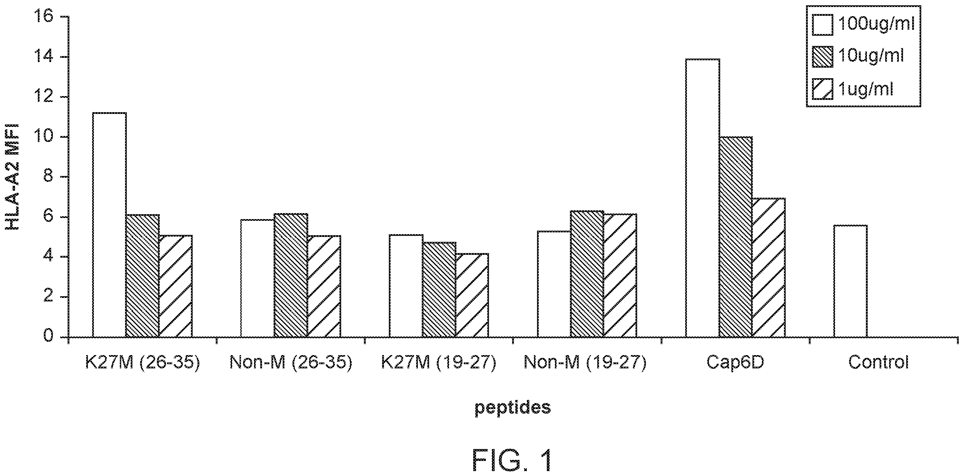

Figure 1. Identification of a HLA-A*0201-restricted epitope in H3.3 with the K27M mutation. A. HLA-A201 binding ability of H3.3-derived peptides was analyzed by T2 cell A2-binding assay. Cap1-6D is an altered peptide ligand, which has been derived from an epitope in human carcinoembryonic Ag, CEA605-613 and was used as positive control. B. H3.3 tetramer staining analysis of the CD8+ CTLs generated with the H3.3.K27M (26-35) after 1st antigen stimulation (left) and weekly re-stimulations (right).

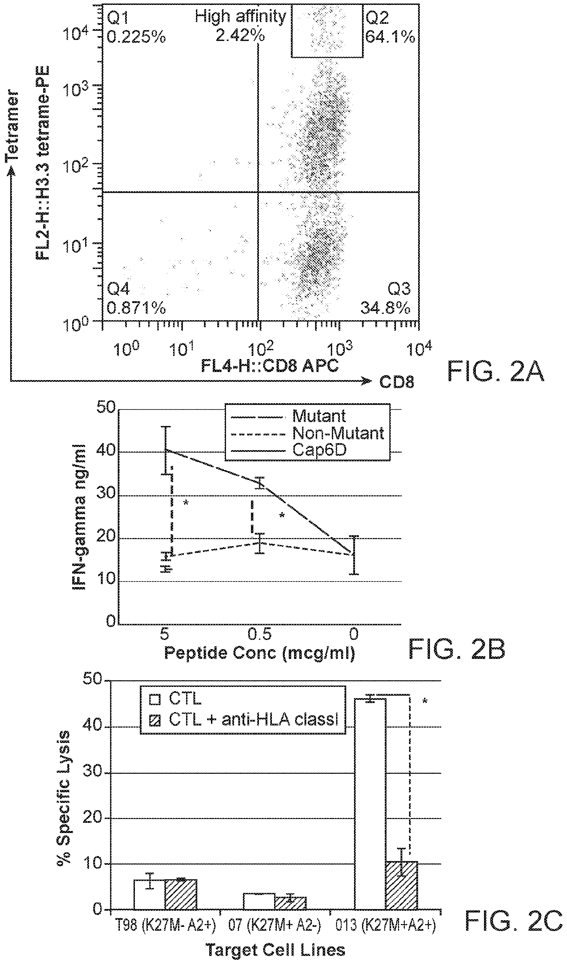

Figures 2A-C. HLA-A*0201+ donor-derived CTLs specifically recognize HLA-A*0201+ K27M+ glioma cells in an HLA-class I-dependent manner. Peripheral blood mononuclear cells from an HLA-A*0201+ donor were stimulated in vitro with the H3.3.K27M peptide and evaluated for their reactivity against: (1A) HLA-A*0201/H3.3.K27M-specific tetramer and anti-CD8 mAb, and (1B) T2 cells pulsed with the mutant or non-mutated H3.3 peptide by IFN-γ ELISA. In (2A), among the CD8+tetramer+ population (64.1% of total lymphocyte-gated cells), there is a tetramer^{high} subpopulation (2.4% of total lymphocyte-gated cells), some of which were used as CTL clones. In (2B), the Cap1-6D peptide (tested at 5 µg/ml only) is a high avidity HLA-A*0201-binding epitope derived from CEA4 used as an irrelevant negative control. (1C) The CTL line was evaluated for cytotoxicity against glioma cell lines T98 (HLA-A*0201+ but K27M-negative), HSJD-DIPG-07 (HLA-A*0201-negative but K27M+), and HSJD-DIPG-13 (HLA-A*0201+ and K27M+) lines. CFSE-labeled target cells (10e4/well) were incubated with CTLs at the E/T ratio of 25 for 4 hours. To block the CTL cytotoxicity, anti-HLA-ABC 10µg/ml was added to one group. At the end of incubation, 7-ADD was added into each well and incubated for 10 minutes on ice. The samples were analyzed by flow cytometry, and the killed target cells were identified as CFSE+ and 7-ADD+ cells. The cytotoxicity was calculated as the percentage of CFSE+ and 7-ADD+ cells in total HLA-A*0201+ CFSE+ cells. (*p<0.05 by Wilcoxon rank-sum tests).

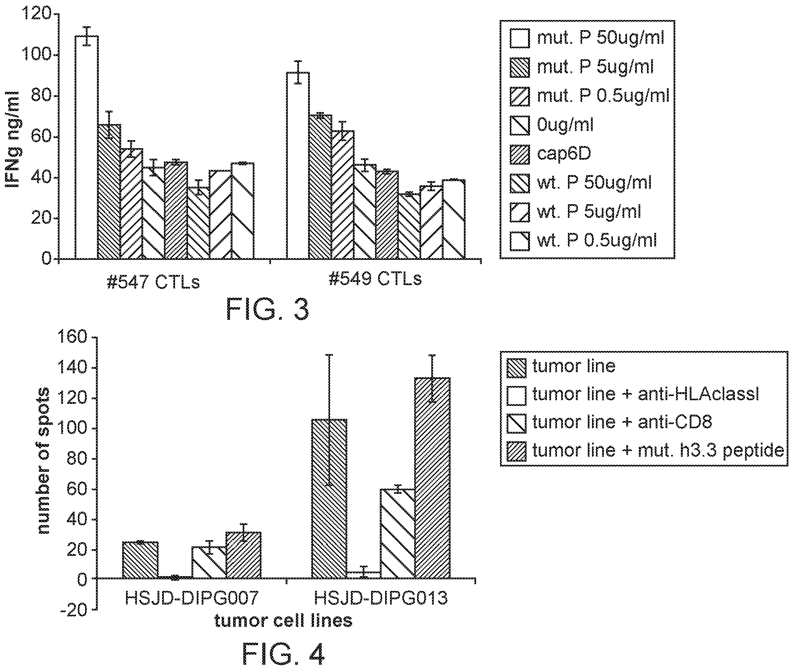

Figure 3. CD8+ T cell lines stimulated with the H3.3.K27M (26-35) peptide recognize the H3.3.K27M (26-35) but not the non-mutant counterpart. CD8+ T cell lines were induced and expanded with the H3.3.K27M (26-35) peptide from the PBMCs derived from two HLA-A*0201+ healthy donors (#547 and #549). T2 cells pulsed with the mutant H3.3.K27M (26-35) peptide or the non-mutant H3.3.non-M (26-35) peptide at the indicated concentrations were mixed with the CD8+ T cell lines for 24 hours. IFN-γ in the supernatants were assessed by ELISA.
Figure 4. HLA-A2+ donor-derived CTLs specifically recognize HLA-A2+ K27M+ glioma cells and secrete IFN-γ in an HLA-A2- and CD8- dependent manners. Peripheral blood mononuclear cells from an HLA-A2 donor were stimulated in vitro with synthetic peptides for H3.3.K27M (26-35) and evaluated their reactivity against HSID-007 (HLA-A2-negative but K27M+) or HSID-013 (HLA-A2+ and K27M+) lines by IFN-γ ELISPOT assays. Anti-HLA-ABC (10µg/ml) and anti-CD8 antibodies were also used to evaluate whether the response is dependent on HLA-A2 and CD8+, respectively.

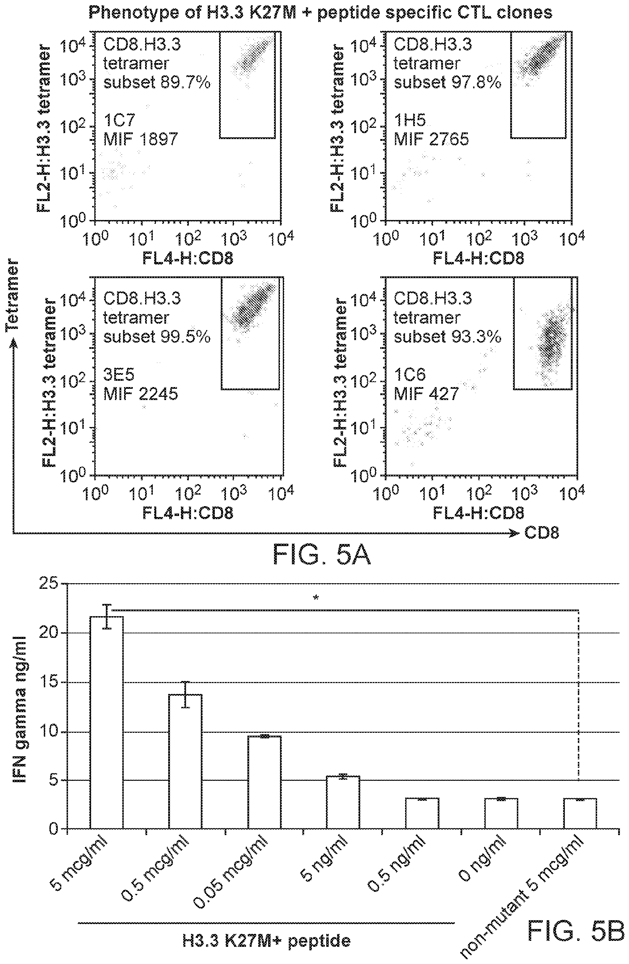

Figure 5A-B. Characterization of H3.3.K27M-specific CTL clones. (5A) Clones were generated by limiting dilution cloning of HLA-A2-H3.3.K27M-tetramer-positive single cells from H3.3 K27M-specific CTLs using FACS-sorting. Clones with relatively high (1C7, IH5 and 3E5) and moderate (1C6) affinity based on the mean fluorescence index (MFI) were selected for further evaluations. (5B) An H3.3.K27M-specific CTL clone (IH5) demonstrates H3.3.K27M-specific reactivity as shown by IFN- γ ELISA against T2 cells pulsed with the mutant H3.3 K27M+ peptide at titrating concentrations and the wild-type peptide (H3.3 K27M-negative; used at 500 ng/ml).

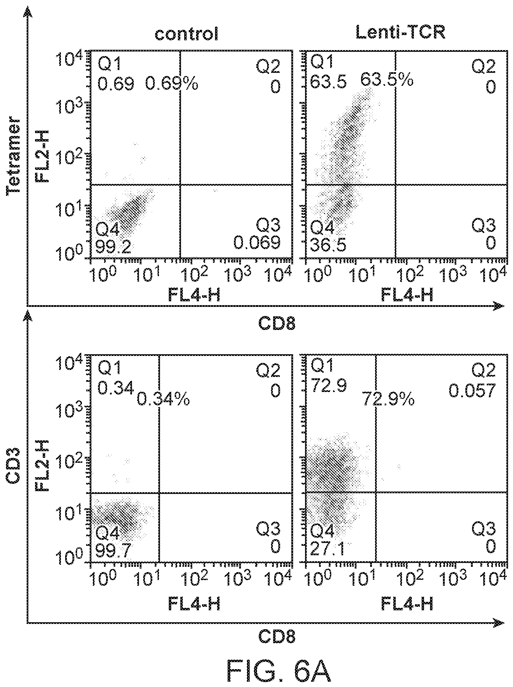

Figure 6A-C. Evaluation of H3.3.K27M-specific TCR. (6A), J.RT-T3.5 cells were transduced with lentiviral vector encoding the TCR α- or β-chains derived from H3.3.K27M-specific CTL clone IH5 (J.RT-T3.5-TCR). The J.RT-T3.5-TCR or control non-transduced J.RT-T3.5 cells were evaluated for the surface TCR expression using PE-labeled HLA-A*0201/ H3.3.K27M tetramer (upper panel) or PE-labeled anti-CD3 mAb (lower panel) and FITC-labeled anti-human CD8 mAb (upper and lower panels). Since J.RT3-T3.5 cells are CD4+ and CD8-negative, tetramer+CD8-negative cells are ones expressing the transgene-derived TCR. CD3-upregulation indicates activation of cells. (6B), J.RT-T3.5-TCR, but not control J.RT-T3.5 cells, upregulate CD69 expression upon recognition of the H3.3 K27M peptide loaded on T2 cells. (3C), DIPG 13 cells [HLA-A*0201+ (albeit dim), K27M mutation+] were incubated with J.RT-T3.5-TCR or control J.RT-T3.5 cells. IL-2 secretion in the culture media was assayed by specific ELISA.

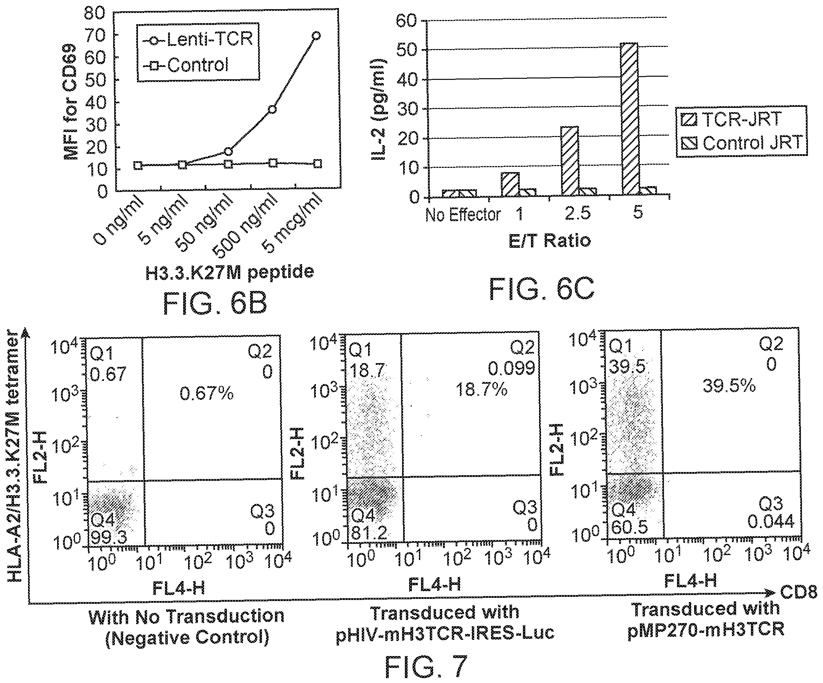

Figure 7. Expression of transgene-derived TCR. J.RT3-T3.5 cells, which are deficient for endogenous TCR β-chain, were transduced with lentiviral vectors (pHIV- mH3TCR-IRES-Luc or pMP270-mH3TCR) encoding TCR α- and β-chains derived from an H3.3.K27M-specific CTL clone (IH5; Figure 7) and evaluated for the surface TCR expression using PE-labeled HLA-A2/ H3.3.K27M tetramer and FITC-labeled anti-human CD8 mAb. Since J.RT3-T3.5 cells are CD4+ and CD8-negative, tetramer+ CD8-negative cells are ones expressing the transgene-derived TCR. Negative control cells are non- transfected cells stained with the same tetramer indicting the specificity of the tetramer-binding.

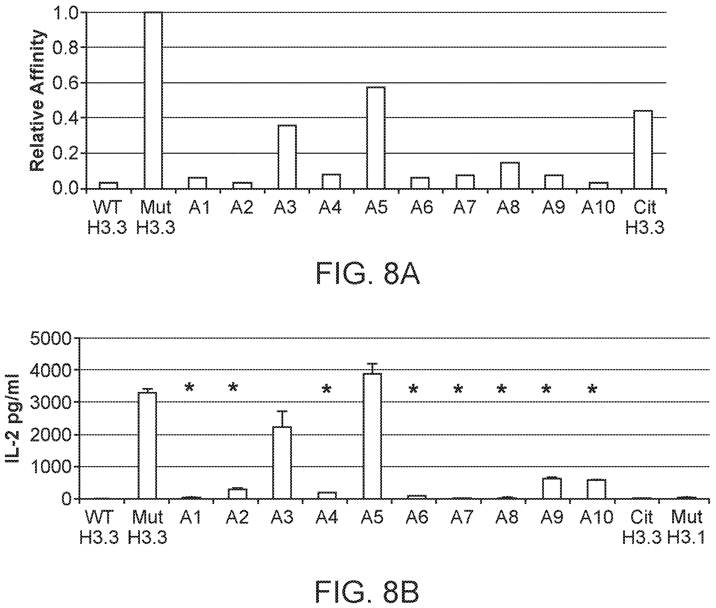

Figure 8A-B. Alanine scanning to determine the key immunogenic AA residues of the H3.3.K27M epitope. (8A) Relative HLA-A2-binding affinity of each peptide to that of H3.3.K27M (26-35) was determined by cell-free binding assay using HLA-A2 purified by affinity chromatography from the EBV transformed homozygous cell line JY. (8B), J.RT3-T3.5 cells were transduced with lentiviral vector encoding the H3.3.K27M-specific TCR and evaluated for the recognition of each peptide loaded on T2 cells by production of IL-2. Each group was assayed as triplicate * <0.05 by Student-t compared with the mutant H.3.3. In addition to 10 synthetic peptides each containing the substitution with alanine (A1-A10), we also evaluated synthetic peptides designed for citrullinated H3.3. K27M epitope (Cit H3.3; *i.e.,* the first AA of the H3.3.K27M epitope is replaced by citrulline) and H3.1 (a homologue ofH3.3.) derived K27M epitope (MutH.3.1).

### DETAILED DESCRIPTION

### Introduction

The inventors have found that a peptide that encompasses amino-acid positions 26-35 of H3.3, which includes the K27M mutation [referred to herein as "H3.3.K27M (26-35)"], can induce specific cytotoxic T lymphocyte (CTL) responses in human leukocyte antigen (HLA)-A2+ donors. Furthermore, CTLs against H3.3.K27M (26-35) recognize HLA-A2+ glioma cell lines that also harbor the K27M mutation. Accordingly, described herein are compositions for use in generating an immune response in human subjects to a peptide comprising amino-acid positions of 26-35 of H3.3 or variants thereof.

### CTL Peptides

The CTL peptides described herein comprise (R/A)MSAP(S/A)TGGV (SEQ ID NO:1), where the amino acid options in parentheses are alternative options for the specified position. Accordingly, CTL peptides can comprise RMSAPSTGGV (SEQ ID NO:4), AMSAPSTGGV (SEQ ID NO:5), RMSAPATGGV (SEQ ID NO:6), or AMSAPATGGV (SEQ ID NO:7). CTL peptides comprising RMSAPSTGGV (SEQ ID NO:4) or AMSAPSTGGV (SEQ ID NO:5) represent peptides for targeting H3.3.K27M (26-35). CTL peptides comprising RMSAPATGGV (SEQ ID NO:6), or AMSAPATGGV (SEQ ID NO:7) represent peptides for targeting the corresponding H3.1 K27M (26-35). The length of the CTL peptides can vary so long as the peptides are effective at inducing an immune response, *e.g.,* a CTL response. The peptide may consist of 100 or fewer or 50 or fewer amino acids. The CTL peptides may have 10-14 amino acids, *i.e.,* may be 10, 11, 12, 13, or 14 amino acids long. As SEQ ID NO:1 is 10 amino acids long, the 11 or 14-amino acid options may have one to four additional amino acids on the amino or carboxyl terminus of SEQ ID NO: 1, or one or two additional amino acid on each of the amino and carboxyl terminus of SEQ ID NO: 1. Additional amino acids can be selected from any of the twenty naturally-occurring amino acids or can be a non- naturally-occurring amino acid.

The peptides can be modified to alter, for example, their in vivo stability. For instance, inclusion of one or more D-amino acids in the peptide typically increases stability, particularly if the D-amino acid residues are substituted at one or both termini of the peptide sequence. Stability can be assayed in a variety of ways such as by measuring the half-life of the proteins during incubation with peptidases or human plasma or serum. A number of such protein stability assays have been described (*see, e.g.,* Verhoef et al, Eur. J. DrugMetab.Pharmacokin. 11:291-302 (1986)).

The peptides can also be modified by linkage to other molecules. For example, different N- or C-terminal groups may be introduced to alter the molecule's physical and/or chemical properties. Such alterations may be utilized to affect, for example, adhesion, stability, bioavailability, localization or detection of the molecules. For diagnostic purposes, a wide variety of labels may be linked to the terminus, which may provide, directly or indirectly, a detectable signal. Thus, the peptides described herein may be modified in a variety of ways for a variety of end purposes while still retaining biological activity.

The following examples of chemical derivatives are described by way of illustration and not by way of limitation.

Aromatic amino acids may be replaced with D- or L-naphylalanine, D- or L-Phenylglycine, D- or L-2-thieneyl-alanine, D- or L-1-, 2-, 3- or 4-pyreneylalanine, D- or L-3-thieneylalanine, D- or L- (2-pyridinyl)-alanine, D- or L- (3-pyridinyl)-alanine, D- or L- (2-pyrazinyl)-alanine, D- or L- (4-isopropyl)-phenylglycine, D-(trifluoromethyl)-phenyl-glycine, D-(trifluoromethyl)-phenylalanine, D-p-fluoro-phenylalanine, D- or L-p-biphenylphenylalanine, D- or L-p-methoxybiphenylphenylalanine, D- or L-2-indole-(alkyl)alanines, and D- or L-alkylamines where alkyl may be substituted or unsubstituted methyl, ethyl, propyl, hexyl, butyl, pentyl, iso-propyl, iso-butyl, sec-isotyl, iso-pentyl, non-acidic amino acids, of C1-C20.

Acidic amino acids can be substituted with non-carboxylate amino acids while maintaining a negative charge, and derivatives or analogs thereof, such as the non-limiting examples of (phosphono) -alanine, glycine, leucine, isoleucine, threonine, or serine; or sulfated (*e.g.,* -SO₃H) threonine, serine, tyrosine.

Other substitutions may include unnatural hyroxylated amino acids made by combining "alkyl" (as defined and exemplified herein) with any natural amino acid. Basic amino acids may be substituted with alkyl groups at any position of the naturally occurring amino acids lysine, arginine, ornithine, citrulline, or (guanidino) -acetic acid, or other (guanidino) alkyl-acetic acids, where "alkyl" is define as above. Nitrile derivatives (*e*.*g*., containing the CN-moiety in place of COOH) may also be substituted for asparagine or glutamine, and methionine sulfoxide may be substituted for methionine. Methods of preparation of such peptide derivatives are well known to one skilled in the art.

In addition, any amide linkage can be replaced by a ketomethylene moiety, *e.g,* (-C(=O) -CH₂-) for (- (C=O) -NH-) . Such derivatives are expected to have the property of increased stability to degradation by enzymes, and therefore possess advantages for the formulation of compounds which may have increased in vivo half-lives, as administered by oral, intravenous, intramuscular, intraperitoneal, topical, rectal, intraocular, or other routes.

In addition, any amino acid can be replaced by the same amino acid but of the opposite chirality. Thus, any amino acid naturally occurring in the L-configuration (which may also be referred to as the R or S configuration, depending upon the structure of the chemical entity) may be replaced with an amino acid of the same chemical structural type, but of the opposite chirality, generally referred to as the D- amino acid but which can additionally be referred to as the R- or the S-, depending upon its composition and chemical configuration. Such derivatives have the property of greatly increased stability to degradation by enzymes, and therefore are advantageous in the formulation of compounds which may have longer in vivo half-lives, when administered by oral, intravenous, intramuscular, intraperitoneal, topical, rectal, intraocular, or other routes.

Fusion peptides including an antigenic peptide as described above fused to another peptide sequence are specifically contemplated for use with the methods and compositions described herein. Peptides include fusion peptides may be composed of a CTL sequence as described herein (*e*.*g*., SEQ ID NO: 1) and a helper T lymphocyte (CD4) epitope sequence fused together. Examples of suitable CD4 epitopes include the synthetic sequence PADRE, tetanus-specific peptides, peptides derived from the same antigen or other antigens from the virus that is to be targeted. Similarly, for cancer antigens, a CD4 peptide derived from the same antigen, or any other cell-antigen known in the art, and the like may be used. Linker peptide sequences at the N- or C-terminal end of the fusion or between the CTL and CD4 epitopes in the fusion also may be used. Such linker sequences generally can be, for example, from about 1 to about 10 amino acids in length and, *e.g,* about 2 to about 7 amino acids or from about 3 to about 5 amino acids in length can optionally comprise modified or non-traditional amino acids.

Also described are peptide/HLA-A2 multimers, and their use to isolate peptide-specific CTLs. "Multimers" include, *e*.*g*., tetramers, pentamers and any number of MHC-peptide structure assembled around the core molecule with fluorochrome. The selected MHC molecules (*e*.*g*., HLA-A2) along with beta2-microgloblin may be assembled around one core molecule which connects a fluorochrome molecule (such as FITC, so that the multimer will fluoresce) and multiple MHC-beta- microgloblin molecules (in case of tetramer and pentamer, there will be 4 and 5, respectively). Then, the MHC molecule is also bound with the peptide so that the MHC-peptide complex on the multimer molecule can be recognized by the TCR on T-cells. *See, e.g.,* Wooldridge, et al, Immunology 126(2): 147-164 (2009); Yokouchi, et al.. Cancer Sci. 97(2): 148-54 (2006). Accordingly, fluorochrome-conjugated peptide-major histocompatibility complex (pMHC) multimers, wherein the peptide is a CTL peptide (*e.g.*, comprising SEQ ID NO: 1) are described.

T-cell populations may be enriched for T-cells expressing one or more TCRs that bind to the CTL peptide/MHC complex. For example, a T-cell population may be cultured in the presence of the CTL peptide (either the naked peptide or peptide loaded onto APCs), thereby preferentially stimulating division of T- cells carrying TCRs that bind the peptide/MHC complex. The culturing occurs in the presence of IL-2, IL-4, IL-7 or IL-15 alone, or of 2-way, 3-way, or 4-way combinations thereof. T-cell populations expanded in this way will be enriched for TCRs that bind the protein/MHC complex. Subsequently, fluorochrome-conjugated peptide-major histocompatibility complex (pMHC) multimers can be used to label the T-cells expressing the TCRs that bind the peptide/MHC complex, and can be sorted, for example by FACS.

Exemplary TCR alpha and beta chain sequences that recognize the H3.3 .K27M epitope are described below with CDR1, CDR2, and CDR3 underlined.
**TCRA-Va19*01/J43** 
Met L T A S L L R A V I A S I C V V S S Met A Q K V T Q A Q T E I S V V E K E D V T
L D C V Y E T R D T T Y Y L F W Y K Q P P S G E L V F L I R R N S F D E Q N E I S G
R Y S W N F Q K S T S S F N F T I T A S Q V V D S A V Y F C A L S E E N D Met R F G
A G T R L T V K P N I Q N P D P A V Y Q L R D S K S S D K S V C L F T D F D S Q T N
V S Q S K D S D V Y I T D K T V L D Met R S Met D F K S N S A V A W S N K S D F A
C A N A F N N S I I P E D T F F P S P E S S C D V K L V E K S F E T D T N L N F Q N L
S V I G F R I L L L K V A G F N L L Met T L R L W S S Stop (SEQ ID NO:8)
CDR1: T R D T T Y Y (SEQ ID NO: 12);
CDR2: RN S F (SEQ ID NO: 13)
CDR3: A L S E (SEQ ID NO: 14)
**Coding sequence of the above amino acid sequence:** 
ATGCTGACTGCCAGCCTGTTGAGGGCAGTCATAGCCTCCATCTGTGTTGTATCCAG
CATGGCTCAGAAGGTAACTCAAGCGCAGACTGAAATTTCTGTGGTGGAGAAGGAG
GATGTGACCTTGGACTGTGTGTATGAAACCCGTGATACTACTTATTACTTATTCTGG
TACAAGCAACCACCAAGTGGAGAATTGGTTTTCCTTATTCGTCGGAACTCTTTTGA
TGAGCAAAATGAAATAAGTGGTCGGTATTCTTGGAACTTCCAGAAATCCACCAGTT
CCTTCAACTTCACCATCACAGCCTCACAAGTCGTGGACTCAGCAGTATACTTCTGT
GCTCTGAGTGAGGAGAATGACATGCGCTTTGGAGCAGGGACCAGACTGACAGTAA
AACCAAATATCCAGAACCCTGACCCTGCCGTGTACCAGCTGAGAGACTCTAAATCC
AGTGACAAGTCTGTCTGCCTATTCACCGATTTTGATTCTCAAACAAATGTGTCACA
AAGTAAGGATTCTGATGTGTATATCACAGACAAAACTGTGCTAGACATGAGGTCT
 
ATGGACTTCAAGAGCAACAGTGCTGTGGCCTGGAGCAACAAATCTGACTTTGCAT
GTGCAAACGCCTTCAACAACAGCATTATTCCAGAAGACACCTTCTTCCCCAGCCCA
GAAAGTTCCTGTGATGTCAAGCTGGTCGAGAAAAGCTTTGAAACAGATACGAACC
TAAACTTTCAAAACCTGTCAGTGATTGGGTTCCGAATCCTCCTCCTGAAAGTGGCC
GGGTTTAATCTGCTCATGACGCTGCGGCTGTGGTCCAGCTGA(SEQ ID NO:9)
**TCRB-Vb27/J2.7** 
Met G P Q L L G Y V V L C L L G A G P L E A Q V T Q N P R Y L I T V T G K K L T V
T C S Q N Met N H E Y Met S W Y R Q D P G L G L R Q I Y Y S Met N V E V T D K G
D V P E G Y K V S R K E K R N F P L I L E S P N P N Q T S L Y F C A S G W G G P F Y
E Q Y F G P G T R L T V T E D L K N V F P P E V A V F E P S E A E I S H T Q K A T L
V C L A T G F Y P D H V E L S W W V N G K E V H S G V S T D P Q P L K E Q P A L
N D S R Y C L S S R L R V S A T F W Q N P R N H F R C Q V Q F Y G L S E N D E W T
Q D R A K P V T Q I V S A E A W G R A D C G F T S E S Y Q Q G V L S A T I L Y E I L
L G K A T L Y A V L V S A L V L Met A Met V K R K D S R G Stop (SEQ ID NO:10)
CDR1: N Met N H E Y (SEQ ID NO: 15);
CDR2: Y S Met N V E V T (SEQ ID NO: 16)
CDR3: CAS G W G G P F Y E Q Y (SEQID NO:17)
**Coding sequence of the above amino acid sequence:**

ATGGGCCCCCAGCTCCTTGGCTATGTGGTCCTTTGCCTTCTAGGAGCAGGCCCCCTGGA
AGCCCAAGTGACCCAGAACCCAAGATACCTCATCACAGTGACTGGAAAGAAGTTAACAG
TGACTTGTTCTCAGAATATGAACCATGAGTATATGTCCTGGTATCGACAAGACCCAGGGC
TGGGCTTAAGGCAGATCTACTATTCAATGAATGTTGAGGTGACTGATAAGGGAGATGTTC
CTGAAGGGTACAAAGTCTCTCGAAAAGAGAAGAGGAATTTCCCCCTGATCCTGGAGTCG
CCCAACCCCAACCAGACCTCTCTGTACTTCTGTGCCAGCGGCTGGGGTGGTCCATTCTA
CGAGCAGTACTTCGGGCCGGGCACCAGGCTCACGGTCACAGAGGACCTGAAAAAC
GTGTTCCCACCCGAGGTCGCTGTGTTTGAGCCATCAGAAGCAGAGATCTCCCACAC
CCAAAAGGCCACACTGGTATGCCTGGCCACAGGCTTCTACCCCGACCACGTGGAG
CTGAGCTGGTGGGTGAATGGGAAGGAGGTGCACAGTGGGGTCAGCACAGACCCGC
AGCCCCTCAAGGAGCAGCCCGCCCTCAATGACTCCAGATACTGCCTGAGCAGCCG
CCTGAGGGTCTCGGCCACCTTCTGGCAGAACCCCCGCAACCACTTCCGCTGTCAAG
TCCAGTTCTACGGGCTCTCGGAGAATGACGAGTGGACCCAGGATAGGGCCAAACC
CGTCACCCAGATCGTCAGCGCCGAGGCCTGGGGTAGAGCAGACTGTGGCTTCACC
TCCGAGTCTTACCAGCAAGGGGTCCTGTCTGCCACCATCCTCTATGAGATCTTGCT
AGGGAAGGCCACCTTGTATGCCGTGCTGGTCAGTGCCCTCGTGCTGATGGCCATGG
TCAAGAGAAAGGATTCCAGAGGCTAG (SEQ ID NO: 11)

### Polynucleotides

Polynucleotides encoding the CTL peptides described herein are described and are referred to as "CTL polynucleotides". The CTL polynucleotides can be a DNA or RNA sequence. The CTL polynucleotide can be operably linked to some or all of transcriptional and translational regulatory elements, such as a promoter, enhancer and polyadenylation sequence. Regulatory sequences are art-recognized and are described, *e.g.,* in Goeddel; Gene Expression Technology: Methods in Enzymology 185, Academic Press, San Diego, CA (1990). The promoter may be a constitutive promoter, *e.g.,* a strong viral promoter, *e.g.,* CMV promoter. The promoter can also be cell- or tissue-specific, that permits substantial transcription of the DNA only in predetermined cells, *e.g.,* in antigen presenting cells, such as the dendritic cell-specific CD1 1c promoter described in Brocker T, J. Leuk. Biology 66:331 -335, 1999. The promoter can also be an inducible promoter, for example, a metallothionein promoter. Other inducible promoters include those that are controlled by the inducible binding, or activation, of a transcription factor, *e.g.,* as described in U.S. Patent Nos. 5,869,337 and 5,830,462 by Crabtree et al*,* describing small molecule inducible gene expression (a genetic switch); International patent applications PCT/US94/01617, PCT/US95/10591, PCT/US96/09948 and the like, as well as in other heterologous transcription systems such as those involving tetracyclin-based regulation reported by Bujard *et al*, generally referred to as an allosteric "off-switch" described by Gossen and Bujard, Proc. Natl. Acad. Sci. U.S.A. (1992) 89:5547 and in U.S. Patents 5,464,758; 5,650,298; and 5,589,362 by Bujard et al. Other inducible transcription systems involve steroid or other hormone-based regulation.

The CTL polynucleotides may also be produced in part or in total by chemical synthesis, *e.g.,* by the phosphoramidite method described by Beaucage and Carruthers, Tetra. Letts., 22:1859-1862 (1981) or the triester method according to the method described by Matteucci et al., J. Am. Chem. Soc, 103:3185 (1981), and may be performed on commercial automated oligonucleotide synthesizers. A double-stranded fragment may be obtained from the single-stranded product of chemical synthesis either by synthesizing the complementary strand and annealing the strand together under appropriate conditions or by adding the complementary strand using DNA polymerase with an appropriate primer sequence.

The CTL polynucleotide operably linked to all necessary transcriptional and translational regulation elements can be injected as naked DNA into a subject or contacted *in vitro* with antigen presenting cells. The CTL polynucleotide and regulatory elements may be present in a plasmid or vector. Thus, the CTL polynucleotide can be DNA, which is itself non-replicating, but is inserted into a plasmid, which may further comprise a replicator. The DNA can be a sequence engineered so as not to integrate into the host cell genome. Exemplary vectors are expression vectors, *i.e.,* vectors that allow expression of a nucleic acid in a cell. Exemplary expression vectors are those which contain both prokaryotic sequences, to facilitate the propagation of the vector in bacteria, and one or more eukaryotic transcription units that are expressed in eukaryotic cells.

Alternatively, derivatives of viruses such as the bovine papillomaviras (BPV-1), of Epstein-Barr virus (pHEBo, pREP-derived and p205) can be used for transient expression of proteins in eukaryotic cells. These viruses expressing the CTL polypeptides can be used to infect APCs, which are then administered to patients. For other suitable expression systems for both prokaryotic and eukaryotic cells, as well as general recombinant procedures, *see* Molecular Cloning: A Laboratory Manual, 2nd Ed., ed. by Sambrook, Fritsch and Maniatis (Cold Spring Harbor Laboratory Press: 1989), Chapters 16 and 17.

The CTL polynucleotide may be expressed in a prokaryote. The polypeptide may be expressed in *E. coli.* The CTL polypeptide may be expressed in *Listeria monocytogenes,* which may be attenuated, and which can be administered to a human individual to provide the CTL peptide to the individual. *See, e.g.,* US Patent Publication No. US2013/0259891. The CTL polynucleotide may be expressed in a eukaryotic cell. For example, the CTL polynucleotide and polypeptide can be expressed in yeast, insect cells, animal cells, including mammalian cells, *e*.*g*., human cells.

### Inducing an immune response

The CTL peptides described herein may be capable of inducing an immune response when administered to an animal *(e.g.,* a human). An exemplary immune response may be a CTL response. The CTL peptides, once introduced can be processed and presented to the MHC class I complex of an antigen presenting cell. Human MHC class I complex includes HLA-A, B, and C alleles. The CTL peptides (*e.g.,* SEQ ID NO:1) have higher affinity to HLA-A2+individuals. See Table 1. The MHC class I-bound epitope is then transported to the cell surface and recognized by cytotoxic T lymphocytes (CTLs) through T cell receptors (TCRs) located on their surface. Recognition of an antigen/MHC complex by the TCR triggers a cascade of protein and cytokine interactions leading to, among other interactions, the activation, maturation and proliferation of the precursor CTLs, resulting in CTL clones capable of destroying the cells exhibiting CTL peptides recognized as foreign.

One or more CTL peptides as described herein may be administered to the patient. One or more polynucleotides encoding one or more CTLs may be introduced to APCs, and these APCs expressing CTLs may be administered into a human patient to induce an immune response, the cytotoxic T cell response. The CTL peptides may be loaded onto the APCs without expressing the CTL peptides in the cell. *See, e.g.,* US Patent No. 8,652,462. The APCs may be harvested from an individual, loaded with the CTL peptides (or the CTL polynucleotides are introduced into the APCs), and then the APCs may be introduced back into the individual (*i.e*., the cells are autologous) and a CTL immune response may be induced in the individual to the CTL polypeptide.

An immune response may be induced by directly administering the CTL peptides to patients. Adjuvants and/or nonspecific inflammatory mediators are not required, but can be optionally administered with the active ingredient before, during, or after the priming and/or boosting of the immune response. Adjuvants are substances that are used to specifically or nonspecifically potentiate an antigen-specific immune response, perhaps through activation of antigen presenting cells. The adjuvant can be, *e.g.,* Montanide-ISA 51 (*e.g.*, from Seppic Hiltonol (*e*.*g*., from Oncovir), anti-CD40 agonistic monoclonal antibodies, polylCLC, Bacillus Calmette-Guerin (BCG) vaccine, an ADP-ribosylating exotoxin (*e*.*g*., cholera toxin, diphtheria toxin, E. coli heat-labile enterotoxin, pertussis toxin, *P. aeruginosa* exotoxin A), a fragment thereof containing the A and/or B subunit, a chemically modified or genetically mutated derivative thereof, or a derivative thereof with reduced toxicity; a chemical conjugate or genetic recombinant containing a bacterial ADP-ribosylating exotoxin or derivative thereof; a chemokine (*e*.*g*., defensins, HCC-1, HCC-4, MCP-1 MCP-3, MCP-4, MLP-1α, MIP-1β, MIP-1γ, MIP-3α, MIP-2, RANTES); another ligand of a chemokine receptor (*e.g*, CCR1, CCR-2, CCR-5, CCR-6, CXCR-1); a cytokine (*e.g.*, IL-1β, IL-2, IL-6, IL-8, IL-10, IL-12; IFN-γ; TNF-α; GM-CSF); another ligand of a cytokine receptor; a salt (*e*.*g*., aluminum hydroxide or phosphate, calcium phosphate); lipid A or a derivative thereof (*e*.*g*., monophosphoryl or diphosphoryl lipid A, lipid A analogs, AGP, AS02, AS04, DC-Choi, Detox, OM-174); a pathogen-associated molecular pattern (PAMP); immunostimulatory CpG motifs in bacterial DNA or an oligonucleotide (see, for example, U.S. Patent 6,218,371); a *Leishmania* homolog of elF4a or a derivative thereof (see, for example, U.S. Patent 5,876,735); a heat shock protein or derivative thereof; C3d tandem array; a muramyl dipeptide (MDP) or a derivative thereof (*e.g.,* murabutide, threonyl-MDP, muramyl tripeptide); ISCOMS and saponins (*e.g.,* Quil A, QS-21); squalene; superantigens; a ligand of a toll-like receptor, or the like. Adjuvants may be chosen to preferentially induce antibody or cellular effectors, specific antibody isotypes (*e*.*g*., IgM, IgD, IgAl, IgA2, secretory IgA, IgE, IgGl, IgG2, IgG3, and/or IgG4), or specific T-cell subsets (*e*.*g*., CTL, Thl, Th2 and/or TDTH)- For example, antigen presenting cells may present Class Il-restricted antigen to precursor CD4+ T cells, and the Th1 or Th2 pathway may be entered.

Described herein is a method of administering APC cells expressing and/or presenting CTL peptides to induce immune response. Methods for delivering CTL polynucleotide to APCs are well-known in the art, for example, retrovimses, adenoviruses, lentiviruses, adeno-associated virus (AAV), and herpes simplex virus-1, vaccinia viruses, and canarypox or fowlpox viruses can infect APC. Most of these vectors cause transient gene expression lasting less than two weeks (Bonnet et al, 2000). To initiate an immune response, expression of peptides, proteins, or MHC molecules may only need to last about three to ten days (*see*, *e.g.,* U.S. Patents 5,656,465 and 5,833,975). Plasmids may also be used to transfer a gene into the cell by itself or with chemicals that enhance transfection, or via carriers. For example, cationic lipids, calcium phosphate, DEAE-dextran, polybrene-DMSO, or polycation amino acids (*e*.*g*., polylysine) are chemical transfectants.

The method may further involve a targeting molecule that preferentially binds to a target cell, for example antigen presenting cells. Such targeting mechanisms may include terminal galactosyl residues binding to asialoglycoprotein receptor (*e*.*g*., galactosylated nucleic acid and/or antigen), high-mannose oligosaccharide binding to mannose receptor (*e*.*g*., mannosylated nucleic acid and/or antigen), ligand binding to an Fc receptor (*e*.*g*., nucleic acid and/or antigen fused or linked to IgG constant region or other ligand of CD64), or membrane proteins highly expressed on antigen presenting cells (*e.g.,* nucleic acid and/or antigen fused or linked to ligand or antibody specific for the membrane protein). Mannose receptors are exemplary targets because they are highly expressed on dendritic cells (especially Langerhans cells), and involved in antigen uptake. This significantly increases the APC's ability to capture exogenous proteins and process them (Sallusto, 1995). Antigen may be delivered to phagocytic cells of the skin such as, for example, Langerhans cells, other dendritic cells, macrophages, and other antigen presenting cells in the epidermis and dermis; antigen may also be delivered to phagocytic cells of the liver, spleen, and bone marrow that are known to serve as the antigen presenting cells through the blood stream or lymphatic system.

The composition can comprise the CTL polypeptide and also comprise the universal T cell epitope called PADRE^{®} (Epimmune, San Diego; described, for example in US Patent No. 5,736,142 or International Application WO95/07707). A "PanDR binding peptide" or "PADRE^{®} peptide" is a member of a family of molecules that binds more than one HLA class II DR molecule. The pattern that defines the PADRE^{®} family of molecules can be thought of as an HLA Class U supermotif. PADRE^{®} binds to most HLA-DR molecules and stimulates in vitro and in vivo human helper T lymphocyte (HTL) responses. Alternatively, T-helper epitopes can be used from universally used vaccines such as tetanus toxoid.

Typically, a vaccine or vaccine composition is prepared as an injectable, either as a liquid solution or suspension. Injection may be subcutaneous, intramuscular, intravenous, intraperitoneal, intrathecal, intradermal, intraepidermal, or by "gene gun". Other types of administration comprise electroporation, implantation, suppositories, oral ingestion, enteric application, inhalation, aerosolization or nasal spray or drops. Solid forms, suitable for dissolving in or suspension in, liquid vehicles prior to injection may also be prepared. The preparation may also be emulsified or encapsulated in liposomes for enhancing adjuvant effect.

A liquid formulation may include oils, polymers, vitamins, carbohydrates, amino acids, salts, buffers, albumin, surfactants, or bulking agents. Exemplary carbohydrates include sugar or sugar alcohols such as mono-, di-, or polysaccharides, or water-soluble glucans. The saccharides or glucans can include fructose, dextrose, lactose, glucose, mannose, sorbose, xylose, maltose, sucrose, dextran, pullulan, dextrin, alpha- and beta- cyclodextrin, soluble starch, hydroxethyl starch and carboxymethylcellulose, or mixtures thereof. "Sugar alcohol" is defined as a C4 to C8 hydrocarbon having an -OH group and includes galactitol, inositol, mannitol, xylitol, sorbitol, glycerol, and arabitol. These sugars or sugar alcohols mentioned above may be used individually or in combination. There is no fixed limit to the amount used as long as the sugar or sugar alcohol is soluble in the aqueous preparation. The sugar or sugar alcohol concentration may be between 1.0 % (w/v) and 7.0 % (w/v), *e*.*g*., between 2.0 and 6.0 % (w/v). Exemplary amino acids include levorotary (L) forms of carnitine, arginine, and betaine; however, other amino acids may be added. Exemplary polymers include polyvinylpyrrolidone (PVP) with an average molecular weight between 2,000 and 3,000, or polyethylene glycol (PEG) with an average molecular weight between 3,000 and 5,000. One can use a buffer in the composition to minimize pH changes in the solution before lyophilization or after reconstitution. Any physiological buffer may be used, but in some cases can be selected from citrate, phosphate, succinate, and glutamate buffers or mixtures thereof. Surfactants that can be added to the formulation are shown in EP patent applications No. EP 0 270 799 and EP 0 268 110.

Additionally, polypeptides can be chemically modified by covalent conjugation to a polymer to increase their circulating half-life, for example. Exemplary polymers, and methods to attach them to peptides, are shown in U S. Patent Nos. 4,766,106; 4,179,337; 4,495,285; and 4,609,546. Exemplary polymers are polyoxyethylated polyols and polyethylene glycol (PEG). PEG is soluble in water at room temperature and has the general formula: R(O-CH₂-CH₂)ₙO-R where R can be hydrogen, or a protective group such as an alkyl or alkanol group. The protective group may have between 1 and 8 carbons, *e*.*g*., methyl. The symbol n is a positive integer, preferably between 1 and 1000, *e*.*g*., between 2 and 500. The PEG can have, for example, average molecular weight between 1000 and 40,000, *e*.*g*., between 2000 and 20,000, *e.g.,* between 3,000 and 12,000. PEG may have at least one hydroxyl group, *e.g.,* it has a terminal hydroxy group.

Water soluble polyoxy ethylated polyols are also useful and can be linked to the CTL polypeptides described herein. They include polyoxyethylated sorbitol, polyoxyethylated glucose, polyoxyethylated glycerol (POG), etc. Another drug delivery system for increasing circulatory half-life is the liposome. The peptides and nucleic acids described herein may also be administered via liposomes, which serve to target a particular tissue, such as lymphoid tissue, or to target selectively infected cells, as well as to increase the half-life of the peptide and nucleic acids composition. Liposomes include emulsions, foams, micelles, insoluble monolayers, liquid crystals, phospholipid dispersions, lamellar layers and the like. Liposomes either filled or decorated with a desired peptide or nucleic acids described herein can be directed to the site of lymphoid cells, where the liposomes then deliver the peptide and nucleic acids compositions. Liposomes for use are formed from standard vesicle-forming lipids, which generally include neutral and negatively charged phospholipids and a sterol, such as cholesterol. The selection of lipids is generally guided by consideration of, *e.g.,* liposome size, acid lability and stability of the liposomes in the blood stream. A variety of methods are available for preparing liposomes, as described in, *e.g.,* Szoka *et al.,* 1980, and U.S. Patent Nos. 4,235,871, 4,501,728, 4,837,028, and 5,019,369.

For targeting cells of the immune system, a ligand to be incorporated into the liposome can include, *e*.*g*., antibodies or fragments thereof specific for cell surface determinants of the desired immune system cells. A liposome suspension containing a peptide may be administered intravenously, locally, topically, etc., in a dose which varies according to, inter alia, the manner of administration, the peptide being delivered, and the stage of the disease being treated.

After the liquid pharmaceutical composition is prepared, the composition can be lyophilized to prevent degradation and to preserve sterility. Just prior to use, the composition may be reconstituted with a sterile diluent (Ringer's solution, distilled water, or sterile saline, for example) which may include additional ingredients. Upon reconstitution, the composition can be administered to subjects.

The polypeptide and APCs described herein can be used to treat individuals having gliomas. The gliomas may be selected from glioblastoma (GBM) and diffuse intrinsic pontine gliomas (DIPG). The individual may be a HLA-A2+ type. The HLA genes are the human versions of the major histocompatibility complex (MHC) genes that are found in most vertebrates (and thus are the most studied of the MHC genes). HLAs corresponding to MHC class I are HLA-A, HLA-B, and HLA-C. HLAs corresponding to MHC class II are DP, DM, DOA, DOB, DQ, and DR. Tests for HLA typing are readily available and can be used to screen patients who are likely benefit from the treatment described herein, for example, from http://labtestsonline.org/understanding/analytes/hla-testing/tab/test/. In some cases, the patient may also have clinical symptoms besides gliomas. The glioma patient may belong to any age group, including children *(e.g.,* 0-18 years old).

### Antibodies recognizing the CTL peptides

Also described is an antibody or an antigen-binding fragment thereof, that recognizes the CTL peptides described herein. Antigen-binding fragments of antibodies may encompass fragments which comprise the hypervariable domains designated CDRs (Complementarity Determining Regions) or part(s) thereof encompassing the recognition site for the antigen, *i.e.,* the CTL peptides described herein, thereby defining antigen recognition specificity. Each Light and Heavy chain (respectively VL and VH) of a four-chain immunoglobulin has three CDRs, designated VL-CDR1, VL-CDR2, VL-CDR3 and VH-CDR1, VH-CDR2, VH-CDR3, respectively. Thus, in some cases the antibody comprises fragments of antibodies of the invention (antigen-binding fragments), which comprise or consist of all or a selection of CDRs among VL-CDR1, VL-CDR2, VL-CDR3 and VH-CDR1, VH-CDR2 and VH-CDR3 or functional portions thereof, *i.e.* portions that exhibit the desired binding specificity, preferably with a high affinity, for the CTL peptides described herein. For example, the antibody or antibody fragment may bind to SEQ ID NO:2 but may not bind to (*e.g.,* binds at no higher than background) SEQ ID NO:3.

The antibody may be produced by any well-known method for antibody production. For example, the antibody may be obtained by administration of any of the CTL peptides described herein into an animal and harvesting the antibodies produced as a result.

### Examples

### Example 1. The H3.3.K27M-derived HLA-A*0201-restricted cytotoxic T-lymphocyte (CTL) epitope and cloning of T-cell receptor (TCR) cDNAs

### 1. Screening of HLA-A*0201-binding epitopes in H3.3 with the K27M mutation

Using the NetMHC 3.4 server (http://www.cbs.dtu.dk/services/NetMHC/), an artificial neural network-based prediction system of peptide binding motifs to HLAs, we predicted that an H3.3-derived 10-mer amino acid (AA) peptide which encompasses the AA position 26-35 including the K27M mutation [H3.3.K27M (26-35)] will serve as a potent epitope in the context of HLA-A*0201, while the non-mutant counterpart for the corresponding positions [H3.3.Non-M (26-35)] was not predicted to have high affinity to HLA-A* 0201 (Table 1).

| **Table 1.** The H3.3 (26-35) peptide | | Binding Scores | logscore | affinity(nM) |
|---|---|---|---|---|
| K27M Mutant: | RMSAPSTGGV | | 0.477 | 285 |
| Non-mutant: | RKSAPSTGGV | | 0.073 | 22651 |

Analysis of H3.3.K27M (26-35), H3.3.Non-M (26-35) peptides as well as 9-mer peptides H3.3.K27M (19-27) and H3.3.Non-M (19-27). We next directly evaluated relative binding affinity of these peptides using the transporter associated with antigen processing (TAP)-deficient HLA-A*0201+ T2 cell line. Since stable binding of HLA-A*0201 with peptide epitopes further stabilizes the surface expression of HLA-A*0201, quantitative expression levels of HLA-A*0201 correlate with the binding affinity of the peptide-epitopes that are co-incubated with the T2 cells **(****Figure 1**). The MFI values for T2 cells with no peptide indicate the baseline HLA-A2 expression level. Cap6D is a well-documented HLA-A*0201-binding epitope and used as a positive control for the assay. The H3.3.K27M (26-35), but none of other H3.3-derived peptides, demonstrated a peptide-dose-dependent increase of mean fluorescence intensity (MFI). These data suggested that H3.3.K27M (26-35) peptide is a potential epitope for specific T-cell responses. To precisely measure the binding of peptides to HLA-A*02:01 class I molecules, we performed a competitive binding inhibition assay, and evaluated the concentration of peptide yielding 50% inhibition of the binding of the radiolabeled probe peptide (IC50). The mutant H3.3.K27M peptide constantly demonstrated IC50 of 95nM-187nM in 4 separate samples, which are considered to be excellent binding capabilities. On the other hand, the non-mutant H3.3 peptide (on the corresponding position) demonstrated >30 fold lower binding capabilities. These data confirm the high HLA A*02:01-binding capability of the H3.3.K27M peptide.

Then, we stimulated HLA-A*0201 donor-derived peripheral blood mononuclear cells (PBMCs) with synthetic peptide for H3.3.K27M (26-35) in vitro for several weekly cycles and evaluated for the induction of CD8+ T-cells capable of binding the HLA-A*0201-H3.3.K27M (26-35) tetramer, and found over 60% of CD8+ cells bound to the tetramer (Figure 2A). Furthermore, a subpopulation of the total CD8+ cells in the culture showed distinctively higher levels of binding to the tetramer, suggesting that these are high-affinity binders among the H3.3.K27M (26-35)-reactive T-cell populations.

### 2. Antigen-specific IFN-γ production and lytic activity of H3.3.K27M (26-35)-stimulated CD8+ T-cells.

CD8+ T cell lines that had been stimulated with the H3.3.K27M (26-35) peptide demonstrate peptide-dose-dependent increases of IFN-γ production in response to T2 target cells loaded with H3.3.K27M (26-35) compared to T2 cells loaded with the control H3.3.Non-M (26-35), cap6D peptide or nothing (Figure 2B and 3).

### 3. H3.3.K27M (26-35)-stimulated CD8+ T-cells recognize the cognate epitope endogenously expressed in HLA-A*0201 glioma cells.

We next examined whether the CD8+ T-cell line developed in response to the H3.3.K27M (26-35) peptide recognize HLA-A*0201+ human glioma cells that endogenously express and present the H3.3.K27M (26-35) epitope. We used HSID-007 (HLA-A*0201-negative but K27M+) or HSID-013 (HLA-A*0201+ and K27M+) as target glioma cells. As illustrated in Figure 2C and Figure 4, the CD8+ T-cell line responded to HSID-013 but not to HSID-007 cells based on cytotoxic T-lymphocyte (CTL) assays (Figure 2C). Furthermore, the observed response against HSID-013 cells was almost completely blocked by anti-HLA-class I blocking antibody. These data are notable because they indicate that the CD8+ T-cell lines (we have similar data for two additional cell lines; but only one is shown here) recognize the H3.3.K27M (26-25) mutant epitope endogenously expressed and presented by HLA-A*0201+ glioma cell lines.

### 4. High affinity H3.3.K27M-specific CTL clones.

We have obtained CTL clones that are reactive to the H3.3.K27M epitope (Figure 5A-B). These clones retain specificity to the H3.3. K27M epitope and we have isolated T-cell receptor (TCR) α and β chains from the clone 1H5.

### 5. Cloning of H3.3.K27M-specific T-cell receptor cDNA.

We have cloned full-length α- (SEQ ID NO:8) and β-chains (SEQ ID NO: 10) of TCRs from an H3.3.K27M-specific CD8+ T-cell clone (1H5), and constructed a lentiviral vector encoding the α- and β-chains. As the first step to confirm expression of the transgene-TCR, we transduced J.RT3-T3.5 cells, which are deficient for endogenous TCR β-chain, with the lentiviral vector and evaluated for expression of HLA-A*0201/H3.3.K27M-tetramer-reactive TCR by flow cytometry (Figure 6A; upper panels; Figure 7). More than 60% of J.RT3-T3.5 cells showed positive tetramer binding, whereas only 0.69% of non-transduced J.RT3-T3.5 cells showed the signal, indicating that the transgene TCR is expressed by both lentiviral constructs. In the same setting, we also observed that TCR-transduced cells, but not control cells upregulate CD3 expression when co-incubated with the tetramer (Figure 6A lower panels). These cells also upregulate CD69 as an indication of activation in a peptide-dose-dependent, and TCR-specific manner (Figure 6B). These data clearly indicate antigen-specific reactivity of the TCR when transduced in J.RT3-T3.5 cells.

Finally, TCR-transduced cells, but not control cells, were able to produce IL-2 when they were co-cultured with HLA-A*0201+, H3.3.K27M+ DIPG-013 cells (Figure 6C).

### 6. Absence of detectable deiminated H3 protein in cultured glioma cells

The natural process of deimination can convert histone arginine to citrulline, including the arginine residue within the H3.3.K27M epitope. Therefore, it is useful to determine whether glioma cells undergo deimination, and, if so, whether the replacement of the arginine residue within the H3.3.K27M epitope with citrulline (*i.e.* deimination) impacts the immunogenicity of the H3.3.K27M epitope.

We performed Western blot analyses to determine whether H3.3.K27M⁺ glioma cells have deiminated H3 protein using a mAb specific to deiminated H3 (abeam cat# ab19847). While neutrophils stimulated with calcium ionophore as the positive control sample showed a single band of approximately 15 kDa corresponding to the deiminated H3, none of the glioma cell lines demonstrated a similar band, strongly suggesting that glioma cell lines are not substantially deiminated, at least when cultured in vitro. The positive control sample is from neutrophils stimulated with calcium ionophore. Glioma cells evaluated are HSJD-DIPG-13, HSJD-DIPG-12, HSJD-DIPG-08, HSJD-DIPG-07, SU06, SU-D-04, T98, and U87. Each lane was loaded with 20 µg protein lysate and high quality SDS-PAGE was confirmed each time by Coomassie Blue staining. We repeated these Western blot analyses at least 4 times (4 SDS-PAGE runs) with different incubation and exposure conditions, and found a thin band with glioma cells at very high exposure conditions (not shown), suggesting that deimination may occur in cultured glioma cells at low levels.

### 7. Alanine scanning data suggests that there are no known human proteins that share the key immunogenic AA residues of the H3.3.K27M epitope

To ensure the specificity and safety of the H3.3.K27M-targeting approach, it is useful to determine key AA residues in the H3.3.K27M epitope that are responsible for the CTL reactivity. This will allow precise predictions and assessments for cross-reactivity to other epitopes derived from proteins in non-tumor normal cells.

To this end, alanine-scanning mutagenesis was used. Single alanine mutations (10 in total) were introduced at every AA residue within the H3.3.K27M decamer (10-mer) epitope. Hence, 10 synthetic peptides each containing the specific substitution with alanine were prepared (A1-A10). Using each of the synthetic peptides with alanine-substitutions, we evaluated whether the substitution alters the stability of peptide-binding to HLA-A*0201 **(****Figure 8A****).** When the binding is diminished by the substitution compared with the natural H3.3.K27M epitope ("Mut H3.3." in **Figure 8A-B**), it indicates that the substituted residue was critical for HLA-A*0201-binding. We also evaluated whether the H3.3.K27M-specific TCR recognizes the altered epitopes presented on T2 cells. To this end, we evaluated IL-2 production as the readout for the activation of TCR-transduced J.RT3-T3.5 cells when co-cultured with T2 cells loaded with each of the altered peptides (**Figure 8B**). AA substitutions that diminish IL-2 production (which was seen with the Mut H3.3 peptide) tell us critical AA for recognition by the TCR. Both binding- **(****Figure 8A****)** and function (*i.e*., IL-2)- **(****Figure 8B****)** based approaches consistently showed that AA at positions 1, 2, 4, 6, 7, 8, 9 and 10 are critical for the recognition. We then carried out an *in silico* search to identify naturally existing AA sequences using NCBI BLAST. We found no known human proteins that contain the same AA residues, indicating that it would be highly unlikely that immunotherapy targeting this epitope will cause unwanted off-target reactions against normal cells.

We also evaluated whether the H3.3.K27M-specific TCR is reactive to the deiminated H3.3K.27M epitope using a synthetic peptide in which the arginine residue of the H3.3.K27M epitope is replaced by citrulline (Cit H3.3) **(****Figure 8A-B****).** While the Cit H3.3 peptide partially retains its affinity to HLA-A*0201 **(****Figure 8A****),** it completely abrogates IL-2 production by TCR-transduced J.RT3-T3.5 cells, indicating that the TCR does not recognize the Cit H3.3 peptide.

A subpopulation of glioma patients bears the K27M mutation in H3.1 (a homologue of H3.3). The AA sequences encompassing the K27M mutation in H3.1 and H3.3 are similar. When compared with the H3.3 .K27M epitope, the corresponding portion of H.3.1 has only one AA substitution. Hence, we evaluated whether the TCR against the H3.3 K27M cross-reacts against H3.1 K27M using a synthetic peptide designed for the putative H3.1 K27M epitope (Mut H3.1 in **Figure 8B****).** The TCR failed to recognize the Mut H.3.1 epitope, suggesting that patients with the H3.1.K27M mutation but without H3.3.K27M mutation are not eligible for prospective TCR-transduced adoptive transfer therapy.

It is understood that the examples and embodiments described herein are for illustrative purposes only and that various modifications or changes in light thereof will be suggested to persons skilled in the art.

## Claims

1. A modified T-cell comprising a T-cell receptor (TCR) specific for a replication-independent histone 3 variant H3.3 or H3.1 peptide comprising the amino acid sequence (R/A)MSAP(S/A)TGGV (SEQ ID NO: 1), wherein the TCR comprises:
(a) a TCR alpha chain, comprising complementarity-determining regions (CDRs) 1, 2, and 3 comprising SEQ ID NOs: 12, 13, and 14, respectively; and
(b) a TCR beta chain, comprising CDRs 1, 2, and 3 comprising SEQ ID NOs: 15, 16, and 17, respectively;
wherein the TCR binds the histone 3 variant H3.3 or H3.1 peptide when the peptide is in a complex with a major histocompatibility complex (MHC).

2. The T-cell according to claim 1, wherein the TCR alpha chain comprises the amino acid sequence set forth in SEQ ID NO: 8, and the TCR beta chain comprises the amino acid sequence set forth in SEQ ID NO: 10.

3. The T-cell according to claim 1 or 2, wherein the TCR is expressed from a lentiviral vector.

4. The T-cell according to any one of claims 1-3, wherein expression of the TCR is under the control of a heterologous promoter, optionally a constitutive promoter.

5. The T-cell according to any one of claims 1-4, wherein the histone 3 variant H3.3 or H3.1 peptide comprises the amino acid sequence RMSAPSTGGV (SEQ ID NO: 2).

6. The T-cell according to any one of claims 1-5, wherein the histone 3 variant H3.3 or H3.1 peptide consists of 10-14 amino acids, optionally 10-12 amino acids.

7. The T-cell according to any one of claims 1-6, wherein the histone 3 variant H3.3 or H3.1 peptide consists of the amino acid sequence SEQ ID NO: 2.

8. The T-cell according to any one of claims 1-7, wherein the R in SEQ ID NO: 1 or 2 is not citrullinated.

9. The T-cell according to any one of claims 1-7, wherein the MHC is HLA-A*0201.

## Patentansprüche

1. Modifizierte T-Zelle, umfassend einen T-Zell-Rezeptor (TCR), der für ein replikationsunabhängiges Peptid der Histon-3-Variante H3.3 oder H3.1, umfassend die Aminosäuresequenz (R/A)MSAP(S/A)TGGV (SEQ ID NO: 1), spezifisch ist, wobei der TCR umfasst:
(a) eine TCR-Alpha-Kette, umfassend die komplementaritätsbestimmenden Regionen (CDRs) 1, 2 und 3, umfassend die SEQ ID NO: 12, 13 beziehungsweise 14; und
(b) eine TCR-Beta-Kette, umfassend die CDRs 1, 2 und 3, umfassend die SEQ ID NO: 15, 16 beziehungsweise 17;
wobei der TCR das Peptid der Histon-3-Variante H3.3 oder H3.1 bindet, wenn das Peptid in einem Haupthistokompatibilitätskomplex (MHC) ist.

2. T-Zelle nach Anspruch 1, wobei die TCR-Alpha-Kette die Aminosäuresequenz umfasst, die in SEQ ID NO: 8 dargelegt ist, und die TCR-Beta-Kette die Aminosäuresequenz umfasst, die in SEQ ID NO: 10 dargelegt ist.

3. T-Zelle nach Anspruch 1 oder 2, wobei der TCR von einem lentiviralen Vektor exprimiert wird.

4. T-Zelle nach einem der Ansprüche 1-3, wobei die Expression des TCR unter der Kontrolle eines heterologen Promotors, optional eines konstitutiven Promotors, erfolgt.

5. T-Zelle nach einem der Ansprüche 1-4, wobei das Peptid der Histon-3-Variante H3.3 oder H3.l die Aminosäuresequenz RMSAPSTGGV (SEQ ID NO: 2) umfasst.

6. T-Zelle nach einem der Ansprüche 1-5, wobei das Peptid der Histon-3-Variante H3.3 oder H3.1 aus 10-14 Aminosäuren, optional 10-12 Aminosäuren, besteht.

7. T-Zelle nach einem der Ansprüche 1-6, wobei das Peptid der Histon-3-Variante H3.3 oder H3.1 aus der Aminosäuresequenz SEQ ID NO: 2 besteht.

8. T-Zelle nach einem der Ansprüche 1-7, wobei das R in SEQ ID NO: 1 oder 2 nicht citrulliniert ist.

9. T-Zelle nach einem der Ansprüche 1-7, wobei die MHC HLA-A*0201 ist.

## Revendications

1. Cellule T modifiée comprenant un récepteur de cellule T (TCR) spécifique d'un peptide de variant H3.3 ou H3.1 d'histone 3 indépendante de la réplication, comprenant la séquence d'acides aminés (R/A)MSAP(S/A)TGGV (SEQ ID N° : 1), dans laquelle le TCR comprend :
(a) une chaîne alpha de TCR, comprenant des régions déterminant la complémentarité (CDR) 1, 2, et 3 comprenant SEQ ID N° : 12, 13, et 14, respectivement ; et
(b) une chaîne bêta de TCR, comprenant des CDR 1, 2, et 3 comprenant SEQ ID N° : 15, 16, et 17, respectivement ;
dans laquelle le TCR lie le peptide de variant H3.3 ou H3.1 d'histone 3 lorsque le peptide est dans un complexe avec un complexe majeur d'histocompatibilité (CMH).

2. Cellule T selon la revendication 1, dans laquelle la chaîne alpha de TCR comprend la séquence d'acides aminés présentée dans SEQ ID N° : 8, et la chaîne bêta de TCR comprend la séquence d'acides aminés présentée dans SEQ ID N° : 10.

3. Cellule T selon la revendication 1 ou 2, dans laquelle le TCR est exprimé à partir d'un vecteur lentiviral.

4. Cellule T selon l'une quelconque des revendications 1 à 3, dans laquelle l'expression du TCR est sous le contrôle d'un promoteur hétérologue, éventuellement d'un promoteur constitutif.

5. Cellule T selon l'une quelconque des revendications 1 à 4, dans laquelle le peptide de variant H3.3 ou H3.1 d'histone 3 comprend la séquence d'acides aminés RMSAPSTGGV (SEQ ID N° : 2).

6. Cellule T selon l'une quelconque des revendications 1 à 5, dans laquelle le peptide de variant H3.3 ou H3.1 d'histone 3 est constitué de 10 à 14 acides aminés, éventuellement de 10 à 12 acides aminés.

7. Cellule T selon l'une quelconque des revendications 1 à 6, dans laquelle le peptide de variant H3.3 ou H3.1 d'histone 3 est constitué de la séquence d'acides aminés SEQ ID N° : 2.

8. Cellule T selon l'une quelconque des revendications 1 à 7, dans laquelle R dans SEQ ID N° : 1 ou 2 n'est pas citrulliné.

9. Cellule T selon l'une quelconque des revendications 1 à 7, dans laquelle le CMH est HLA-A*0201.
